# EUROPÄISCHE PATENTSCHRIFT

(11) **EP 3 865 100 B1**
(45) Veröffentlichungstag und Bekanntmachung des Hinweises auf die Patenterteilung: **19.11.2025**
(21) Anmeldenummer: 21157123.7
(22) Anmeldetag: 15.02.2021
(51) Int. Cl.: A61F 5/01

(54) **DYNAMISCHER STABILITÄTSTRÄGER**
DYNAMIC STABILITY CARRIER
SUPPORT DYNAMIQUE DE STABILITÉ

(30) Priorität: 14.02.2020 DE 102020201886
(43) Veröffentlichungstag der Anmeldung: 18.08.2021
(73) Patentinhaber: Pohlig GmbH, 83278 Traunstein (DE)
(72) Erfinder: Kienzle, Christian, 83064 Raubling (DE); Michalke, Lisa, 83229 Aschau i. Chiemgau (DE); Loidl, Maximilian, 83259 Schleching (DE); Mader, Klaus, 83329 Waging am See (DE); Kapeller, Bruno, 83313 Siegsdorf (DE)
(74) Vertreter: Pfenning, Meinig & Partner mbB

(56) Entgegenhaltungen:
- EP-A1- 3 085 343
- CN-U- 204 121 708
- JP-A- H11 313 862
- US-A1- 2005 209 541
- US-A1- 2005 273 028
- US-A1- 2019 008 671

## Beschreibung

Die vorliegende Erfindung betrifft eine Orthese oder Prothese zur Korrektur einer Fehlstellung eines zwei Glieder verbindenden Gelenks. Solche Orthesen oder Prothesen finden beispielsweise bei der Behandlung eines Klumpfußes oder anderer pathologischer Fehlstellungen Anwendung.

Üblicherweise werden in Orthesen oder Prothesen starre Stabilitätsträger verbaut, die die Beweglichkeit des Fußes sehr stark einschränken. Beispielsweise sind starre Verbindungen von Schalenteilen zur Behandlung von Knöchelgelenken der Standard, wobei diese lediglich die Flexion und Extension des Gelenks ermöglichen.

Orthesen oder Prothesen aus Carbonfaser haben den Nachteil, dass eine gezielte Limitierung der Bewegung nicht möglich ist. So wird durch die Verwendung eines Stabes aus Carbonfaser als Verbindungselement eine seitliche Bewegung (Eversion und Inversion) zusätzlich zur Flexion und Extension zugelassen.

Spiralorthesen bieten dem zu behandelnden Gelenk die Möglichkeit eine Rotationbewegung durchzuführen, allerdings kann dabei die Rotationsbewegung des Gelenks nicht eingeschränkt werden, wodurch auch eine pathologische Bewegung des zu korrigierenden Gelenks zugelassen sein kann, was der Behandlung entgegenwirkt.

Bandagen sind ebenfalls ein probates Mittel zur Behandlung von Fehlstellungen von Gelenken, haben allerdings den Nachteil, dass sie keine festen Bestandteile aufweisen und somit nicht konstant das gleiche Behandlungsergebnis erzielen, da sie sich lösen können oder bei erneuter Anbringung anders angebracht werden.

Eine weitere Behandlungsmethode von Fehlstellungen eines Gelenks besteht im Einsatz von Rotationsstäben, welche die Rotation des Gelenks einschränken können. Der Nachteil dieser Behandlungsmethode liegt allerdings darin, dass nur die Rotationsbewegung eingeschränkt wird, aber keine Limitierung der Flexionsbewegung vorhanden ist, also die Möglichkeit einer pathologischen Flexionsbewegung des zu korrigierenden Gelenks bestehen bleibt.

Eine Orthese oder Prothese des Standes der Technik kann beispielsweise zur Behandlung eines Klumpfußes verwendet werden. In der Behandlung des Standes der Technik wird meist eine Unterschenkelorthese mit einem starren Stabilitätsträger verwendet. Mithilfe einer ringförmigen Fußfassung wird das Subtalargelenk (ein Teilgelenk des Sprunggelenks) bzw. der Calcaneus (das Fersenbein) in einer Valgusposition fixiert. Die calcaneopedale Einheit und der Rotationsvorgang beim Anziehen sorgen für die nötige Eversion (Auswärtsdrehung) im Subtalargelenk und einer Pronation (Einwärtsdrehung) im Mittelfuß. Die Unterschenkelorthese wirkt dabei wie eine externe Arthrodese (Versteifung) auf das Subtalargelenk, wobei die Fußeinheit über einen starren Stabilitätsträger (unilateral oder bilateral) mit der Unterschenkeleinheit fixiert ist. Die konstruierte Kondylenbettung bewirkt eine Rotationsstabilität auf Unterschenkel und Knie und das Bewegungsausmaß des oberen Sprunggelenks ist je nach Befund eingestellt. Der Nachteil dabei ist, dass nicht nur die pathologische sondern auch die physiologische Bewegung eingeschränkt sind.

Definitionen verwendeter anatomischer Fachbegriffe können beispielsweise in Prometheus, LernAtlas der Anatomie, 3. Auflage 2011, Thieme Verlag nachgeschlagen werden.

US 1005/273028 A1 beschreibt eine Knöchel-Fuß-Orthese zum Unterstützen oder Verbessern der Fuß- und Knöchelbewegung eines Benutzers, wobei die Orthese mindestens ein Strebenelement, eine Wadenschale, eine Fußschale und eine Vielzahl von Segmenten umfasst. Zwischen benachbarten Segmenten, einem obersten Segment und der Wadenschale und einem untersten Segment und der Fußschale gebildete Lücken weisen Spaltbreiten auf, wobei Lücken an einer höheren Stelle größere Spaltbreiten aufweisen als solche an einer niedrigeren Stelle. Bei der Plantarflexion nehmen die Lücken in ähnlicher Weise von unten nach oben ab, wodurch die Steifigkeit der Orthese allmählich zunimmt, ein progressiver Plantarflexionsanschlag geschaffen wird und das Ausmaß der auf den Benutzer übertragenen Belastungen abnimmt.

US 2005/209541 A1 beschreibt eine Orthesenvorrichtung zur Wiederherstellung des Bewegungsbereichs einer Gliedmaße eines Patienten, mit einer ersten Vorrichtungsmanschette, die zur Befestigung der Vorrichtung an einem oberen Bereich einer Gliedmaße eines Patienten oberhalb eines Gliedmaßengelenks konfiguriert ist, einer zweiten Vorrichtungsmanschette, die zur Befestigung der Vorrichtung an einem unteren Bereich der Gliedmaße des Patienten unterhalb des Gliedmaßengelenks konfiguriert ist, und einer zwischen der ersten und der zweiten Manschette angebrachten Feder und offenbart die Merkmale des Oberbegriffs des Anspruchs 1.

CN 204 121708 U beschreibt eine Gelenkbewegungsunterstützungs-Schutzvorrichtung, die einen Gelenkschutzkörper, der in seiner Form an ein Gelenk angepasst ist, und eine Befestigungsvorrichtung umfasst, die fest an dem Gelenk eines menschlichen Körpers angebracht ist. Eine Vielzahl von streifenförmigen Befestigungsgurten mit Widerhaken und Schnallen werden für die Befestigungsvorrichtung verwendet. Mehrere obere Kerben, die sich von der Oberseite nach unten erstrecken, aber nicht durch den Boden hindurchgehen, sind in dem Teil des Gelenkschutzkörpers ausgebildet, der sich in der Nähe des Gelenkbewegungsbereichs befindet. Entsprechende untere Aussparungen sind an den Positionen ausgebildet, die sich von der Unterseite aus nach oben erstrecken und den entsprechenden Positionen der oberen Aussparungen entsprechen.

US 2019/008671 A1 beschreibt eine Fingerschiene, die so konfiguriert ist, dass sie um einen Längsabschnitt eines menschlichen Fingers zwischen einem proximalen und einem distalen Ende der Stütze angeordnet werden kann. Wenn sie zur Abstützung installiert ist, ist das proximale Ende zumindest teilweise an der Außenseite des Fingers an einer Stelle hinter dem PIP-Gelenk verankert. Das distale Ende weist einen Schaft auf, der für eine Koextension entlang eines Längsteils der Außenseite des Fingers zwischen dem PIP-Gelenk und dem Fingernagel konfiguriert ist. Zwischen dem proximalen und dem distalen Ende der Stütze befindet sich eine PIP-Gelenkstütze, die das PIP-Gelenk zumindest teilweise umschließt und stützt.

EP 3 085 343 A1 beschreibt eine Orthese zur Beübung eines ein oberes und ein unteres Sprunggelenk aufweisenden menschlichen Sprunggelenks in einer korrigierten Stellung, umfassend einen Fußteil zur Aufnahme eines Fußes, einen Unterschenkelteil zur Aufnahme eines Unterschenkels, wobei der Fußteil und der Unterschenkelteil derart zueinander ausgerichtet sind, dass das Sprunggelenk in eine korrigierte Stellung bringbar ist und mindestens ein erstes Orthesengelenk, wobei das erste Orthesengelenk eine Drehung des Fußteils relativ zum Unterschenkelteil um eine erste Drehachse ermöglicht, wobei die erste Drehachse einer Drehachse des unteren Sprunggelenks in der korrigierten Stellung des Sprunggelenks entspricht.

JP H 11 313862 A beschreibt eine Orthese für untere Gliedmaßen, die ein Fußsohlen-Befestigungselement und ein Waden-Befestigungselement umfasst. Ein ansteigendes, geneigtes Verbindungsteil, das am hinteren Ende des Fußsohlenbefestigungselements ausgebildet ist, und ein abfallendes, geneigtes Verbindungsteil, das am unteren Ende des Wadenbefestigungselements ausgebildet ist, sind durch ein rollengelagertes Verbindungselement drehbar verbunden. Ein Regulierungselement ist über das Fußsohlenbefestigungselement und das Wadenbefestigungselement gelegt, um den Drehwinkel zu regulieren.

Aufgabe der vorliegenden Erfindung ist es somit, eine Orthese oder Prothese zu schaffen, die einerseits steif genug ist, um eine Fehlstellung eines zu behandelnden Gelenks zu korrigieren, indem sie eine pathologische Bewegung einschränkt, aber andererseits flexibel genug um eine physiologische Bewegung zu ermöglichen. Dabei soll die Einschränkung bzw. Möglichkeit der Bewegung einstellbar sein.

Diese Aufgabe wird durch Orthesen oder Prothesen nach Anspruch 1 gelöst. Vorteilhafte Weiterbildungen der erfindungsgemäßen Orthese oder Prothese werden in den abhängigen Ansprüchen gegeben.

Die Nuten sind derart angeordnet, dass sie beabstandet zueinander entlang der Längserstreckung des zumindest einen flachen Stabes verteilt sind. Die Längserstreckung des zumindest einen Stabes ist dabei als diejenige Richtung definiert, die, entlang des flachen Stabes, von der ersten Befestigungsvorrichtung zur zweiten Befestigungsvorrichtung verläuft. Die Nuten verlaufen dabei im Wesentlichen quer zur Längserstreckung des zumindest einen flachen Stabes.

Bevorzugterweise besteht der zumindest eine flache Stab des Verbindungselements der Orthese oder Prothese dabei aus einer, zwei oder mehreren Lagen, wobei sich die eine, zwei oder mehreren Lagen im Wesentlichen parallel zu mindestens einer der Hauptoberflächen des mindestens einen flachen Stabes erstrecken.

Die mehreren Lagen haben dabei den Vorteil, dass zum Beispiel durch Verwendung verschiedener Materialien die Flexibilität bzw. den Elastizitätsmodul des Verbindungselements und somit der Orthese oder Prothese eingestellt werden kann.

Bevorzugterweise hat der flache Stab des Verbindungselements der Orthese oder Prothese eine Breite C mit 5 mm ≤ C ≤ 35 mm, vorteilhafterweise 7 mm ≤ C ≤ 25 mm, vorteilhafterweise 10 mm ≤ C ≤ 22 mm aufweist.

Bevorzugterweise hat der flache Stab des Verbindungselements der Orthese oder Prothese eine Dicke D mit 0,5 mm ≤ D ≤ 15 mm, vorteilhafterweise 1 mm ≤ D ≤ 10 mm, vorteilhafterweise 2 mm ≤ D ≤ 7 mm aufweist.

Bevorzugterweise hat der mindestens eine flache Stab des Verbindungselements eine Länge L mit 20 mm ≤ L ≤ 1000 mm, vorteilhafterweise 30 mm ≤ L ≤ 900 mm, vorteilhafterweise 50 mm ≤ L ≤ 750 mm.

Die Abmessungen des mindestens einen flachen Stabes können dabei an das jeweilige Gelenk und beispielweise mit dem damit verbundenen Abstand zwischen den beiden Befestigungsvorrichtungen angepasst werden. Bei der Behandlung eines Fußgelenks macht es beispielsweise einen Unterschied ob das Gelenk eines Kindes oder eines Erwachsenen behandelt wird, da ein Erwachsener größer und kräftiger ist als ein Kind.

Die Nuten, die am Verbindungselement der Orthese oder Prothese angebracht sind, sind derart ausgestaltet, dass sie mit der Längserstreckungsrichtung des Stabes einen Winkel W von 10 °≤|W|≤90 °, vorteilhafterweise einen Winkel von 30 °≤|W|≤90 °, insbesondere senkrecht zur Längserstreckung verlaufen. Abhängig vom Winkel kann die Elastizität der Orthese oder Prothese in Bezug auf eine Rotationsbewegung verändert werden. Dabei gilt, je kleiner der Winkel W, desto unterschiedlicher ist die Torsion bei Drehung um eine Achse parallel zur Längserstreckungsrichtung im bzw. gegen den Uhrzeigersinn. In dem Fall, in dem die Nuten senkrecht zur Längserstreckungsrichtung ausgerichtet sind, ist die für Torsion des Stabes um die Achse parallel zur Längserstreckungsrichtung in beiden Richtungen gleich, allerdings bestehen zwei unterschiedliche Kräfte für Auslenkungen senkrecht zur Haupterstreckungsebene des zumindest einen Stabes. Für den Fall, dass der Stab in eine Richtung gebogen wird, in der die Nuten zusammengedrückt werden ist signifikant mehr Kraft notwendig als für eine Biegung in eine Richtung in der die Nuten auseinandergezogen werden. Derselbe Effekt kommt auch bei schrägen Nuten vor, ist dort allerdings nicht so ausgeprägt wie bei Nuten, die senkrecht auf der Längserstreckung stehen. Mit der Orientierung der Nut kann zusätzlich auch der Kraftaufwand zur Torsion des Stabes beeinflusst werden.

Die Richtung, in welche die Nuten stehen, kann also dafür verwendet werden die Flexibilität bzw. den Elastizitätsmodul in Bezug auf Torsion und Biegung des Verbindungselements einzustellen und ist somit auf spezifische Krankheitsbilder anpassbar.

Die Nuten im Verbindungselement der Orthese oder Prothese sind derart ausgestaltet, dass sie teilweise nur bereichsweise über eine Richtung quer zur Längserstreckungsrichtung verlaufen. Im Falle eines bereichsweisen Verlaufs kann beispielsweise eine Nut mehrere Bereiche aufweisen, zum Beispiel Vertiefungen an beiden Rändern des Stabes und einem Abschnitt zwischen den beiden Vertiefungen, der nicht vertieft ist. Die Nut kann aber auch in mehr als drei Abschnitte unterteilt werden, wobei sich Vertiefung und Erhebung, bzw. Vertiefungen unterschiedlicher Tiefen, abwechseln. Dabei
muss die Ausdehnung der einzelnen Abschnitte in Richtung entlang der Nut nicht immer gleich sein. So können im vorherigen Beispiel (drei Abschnitte) die Vertiefungen jeweils eine Ausdehnung in Richtung entlang der Nut von 20% der Gesamtlänge der Nut aufweisen und die restlichen 60% aus der Erhebung bestehen.

Der Vorteil einer abschnittsweisen Gestaltung der Nuten ist darin zu sehen, dass dadurch wiederum die Flexibilität des Verbindungselements gestaltet werden kann. Ein geringerer Anteil an Vertiefung führt dabei zu einem Verhalten, das jenem eines durchgehenden Stabes ohne Nuten ähnelt und somit beispielsweise der Unterschied in die beiden Biegerichtungen verringert werden kann. Selbiges kann auch über das Torsionsverhalten gesagt werden.

Bevorzugterweise können die Nuten geradlinig oder in gekrümmter Form verlaufen. So können die Nuten, wenn sie in einer Richtung senkrecht zur Längserstreckungsrichtung verlaufen beispielsweise die Form eines liegenden "S" aufweisen.

Bevorzugterweise weisen die Nuten eine Tiefe T von 0,3 mm ≤ T ≤ 10 mm, vorteilhafterweise 1 mm ≤ T ≤ 6 mm, vorteilhafterweise 2 mm ≤ T ≤ 5 mm auf. Die Tiefe T der Nuten ist dabei in die Richtung definiert, die senkrecht auf die beiden Hauptoberflächen steht und von der einen, die Nut enthaltenden, Hauptoberfläche zur anderen Hauptoberfläche verläuft. Dabei müssen nicht alle Nuten die gleiche Tiefe aufweisen. So können beispielsweise die hälfte der Nuten eine Tiefe von 2 mm und die andere Hälfte eine Tiefe von 3 mm aufweisen. Wie diese Nuten entlang des Stabes verteilt sind ist dabei eben so wenig vorgeschrieben. Es können sich alle 2 mm tiefen Nuten in einer Hälfte des Stabes und alle 3 mm tiefen Nuten in einer anderen Hälfte des Stabes befinden oder die Nuten mit unterschiedlichen Tiefen wechseln sich gegenseitig ab. Weiterhin ist die Anzahl an unterschiedlichen Tiefen nicht auf zwei begrenzt, sondern lediglich durch die Anzahl der Nuten, da alle jeweils eine unterschiedliche Tiefe aufweisen können. Die Tiefe T muss dabei über den Verlauf der Nut hinweg nicht konstant sein, sondern kann sich auch verändern. So ist es beispielsweise möglich, dass die Tiefe der Nut im Verlauf von einer Seite des Stabes hin zu einer anderen Seite des Stabes zu- oder abnimmt. Auch eine Mischung der Zu- und Abnahme der Dicke ist möglich.

Die Tiefe T der Nuten kann dabei wieder dazu verwendet werden, die Eigenschaften des Verbindungselements in Bezug auf Biegung und Torsion desselbigen zu kontrollieren. So entfernen tiefere Nuten mehr Material und können dadurch die Biegeeigenschaften stärker beeinflussen.

Bevorzugterweise weisen die Nuten in dem zumindest einem Verbindungselement der Orthese oder Prothese eine Breite B mit 0,05 mm ≤ B ≤ 10 mm, vorteilhafterweise 0,1 mm ≤ B ≤ 5 mm, vorteilhafterweise 0,1 mm ≤ B ≤ 0,5 mm auf. Die Breite der Nut ist, bei geradlinigem Verlauf der Nut als diejenige Erstreckung in eine Richtung parallel zur die Nut aufweisenden Hauptoberfläche und senkrecht zur Ausbreitungsrichtung der Nut anzusehen. Im Fall einer gekrümmten Nut ist die Breite der Nut als diejenige Erstreckung einer Richtung parallel zur Hauptoberfläche und senkrecht zur örtlichen Verlaufsrichtung der Nut anzusehen. Ähnlich zum Merkmal der Tiefe, können alle Nuten dieselbe Breite B aufweisen, es können aber auch alle Nuten unterschiedliche Breiten haben. Kombinationen aus zwei oder mehreren unterschiedlichen Breiten sind dabei genauso möglich. Dabei ist die Breite jeder Nut nicht auf eine Breite beschränkt, sondern kann sich auch entlang der Erstreckungsrichtung der Nut verändern. Beispielsweise kann sich die Nut im Verlauf von einer Seite des Stabes zur anderen Seite des Stabes verjüngen oder erweitern. In einem anderen Beispiel kann sich die Nut zur Mitte des Stabes hin erweitern und zum gegenüberliegenden Ende hin wieder verjüngen.

Die Breite der Nut kann wiederum dazu verwendet werden die Flexibilität bzw. den Elastizitätsmodul des Verbindungselements unter Einfluss von Torsion oder Biegung zu beeinflussen.

Bevorzugterweise weisen die Nuten in dem zumindest einem Verbindungselement der Orthese oder Prothese in Richtung der Längserstreckung des Stabes zueinander einen Abstand A mit 5 mm ≤ A ≤ 40 mm, vorteilhafterweise 10 mm ≤ A ≤ 35 mm, vorteilhaft-erweise 12 mm ≤ A ≤ 28 mm auf. Der Abstand ist dabei vom Mittelpunkt einer Nut zum Mittelpunkt der nächsten Nut bestimmt. Der Abstand zwischen den einzelnen Nuten kann dabei gleich sein, aber sich auch von Nut zu Nut ändern oder einem vordefinierten Muster folgen. So kann der Abstand A der Nuten beispielsweise abwechselnd 12 mm und 25 mm betragen. Bevorzugterweise ist die Orthese oder Prothese derart ausgestaltet, dass das erste Befestigungselement am Fuß einer Person befestigt ist und das zweite Befestigungselement mit dem Unterschenkel desselben Beines verbunden ist. Das Verbindungselement das zwischen den beiden Befestigungselementen verläuft kann dadurch das Fußgelenk des Beines unterstützen bzw. dessen Position korrigieren, in dem pathologische Bewegungen eingeschränkt werden, während physiologische Bewegungen des zu korrigierenden Gelenks erhalten bleiben.

Der Vorteil einer solchen Anordnung der Orthese oder Prothese ist es, dass dadurch Fehlstellungen des Fußgelenks, wie beispielsweise ein Klumpfuß effektive behandelt werden können.

Bevorzugterweise ist der mindestens eine Stab des Verbindungselements derart positioniert ist, dass die beiden Hauptoberflächen des zumindest einen flachen Stabes mit einer Ebene senkrecht zur Erstreckungsrichtung des ersten Gliedes, bei Rotation um eine Rotationsachse der Flexion des Gelenks, einen Neigungswinkel N einschließen. Der Neigungswinkel N ist dabei 70 °≤ N ≤110 °, vorteilhafterweise 80 °≤ N ≤100 °. Insbesondere können die Hauptoberflächen orthogonal auf die beschriebene Ebene stehen.

Bevorzugterweise schließen die beiden Hauptoberflächen des mindestens einen flachen Stabes mit einer Ebene senkrecht zur Erstreckungsrichtung des ersten Gliedes bei Rotation um eine Achse senkrecht zur Rotationsachse der Flexion des Gelenks und parallel zur Ebene senkrecht zur Erstreckungsrichtung des ersten Gliedes einen Neigungswinkel M ein. Der Neigungswinkel M ist 70 °≤ M ≤110 °, vorteilhafterweise 80 °≤ M ≤ 100, bzw. stehen die beiden Hauptoberflächen insbesondre orthogonal auf die oben beschriebene Ebene.

Bevorzugterweise ist der mindestens eine Stab des Verbindungselements so angebracht, dass die beiden Hauptoberflächen des mindestens einen flachen Stabes mit einer Ebene senkrecht zur Ebene, die durch eine Erstreckungsrichtung des ersten Gliedes und eine Rotationsachse der Flexion des Gelenks aufgespannt ist und parallel zur Erstreckungsrichtung des ersten Gliedes verläuft einen Rotationswinkel R einschließen. Der Rotationswinkel R ist dabei 0 °≤ |R| ≤45 °, vorteilhafterweise 0 °≤ |R| ≤15 °, bzw. sind die beiden Hauptoberflächen des zumindest einen Stabes insbesondere parallel zur oben definierten Ebene.

Die Anpassung der Neigungswinkel N und M der und des Rotationswinkels R der Hauptoberfläche des mindestens einen Stabes des Verbindungselements ermöglicht es, die Orthese oder Prothese an die spezielle Fehlstellung des zu behandelnden Gelenks und an die Physiologie des Beines anzupassen.

Die Nuten sind auf der einen oder den beiden Hauptoberflächen derart angeordnet, dass eine Auslenkung in eine der beiden Richtungen senkrecht zu den beiden im Wesentlichen parallelen Hauptoberflächen einen anderen Elastizitätsmodul aufweist als eine Auslenkung in die entgegengesetzte Richtung. So kann beispielsweise die Bewegung eines Fußgelenks in laterale Richtung durch ein höheres Elastizitätsmodul stärker eingeschränkt sein als eine Bewegung des Fußgelenks in medialer Richtung.

Bevorzugterweise sind die Nuten derart angeordnet, dass die Flexibilität eines Stabes F1 in eine der beiden Auslenkungsrichtungen im Bereich 4 N ≤ F1 ≤ 500 N, vorteilhafterweise 4 N ≤ F1 ≤ 320 N und die Flexibilität eines Stabes F2 bei gleicher Auslenkung im Bereich 30 N ≤ F2 ≤ 6000 N, vorteilhafterweise 48 N ≤ F2 ≤ 3840 N liegt.

Das Elastizitätsmodul und die damit zusammenhängende Flexibilität des mindestens einen Stabes, welches durch die Anordnung der Nuten entlang der Längsrichtung des Stabes eingestellt werden kann, kann dazu verwendet werden, die Bewegungsfreiheit des zu behandelnden Gelenks kontrollieren zu können und somit Orthesen oder Prothesen an die spezifische Fehlstellung anzupassen.

Bevorzugterweise weist die mindestens eine, mehrere oder alle der Nuten einen Querschnitt auf, der rechteckig, insbesondere quadratisch, abgerundet, insbesondere halbrund oder elliptisch, trapezförmig, konisch, dreieckig, vieleckig, rautenförmig und/oder freiförmig ist. Dabei ist, wie schon bei der Breite und Tiefen der Nuten, eine Kombination verschiedener Querschnitte in geordneter Form, beispielsweise abwechselnd ein dreieckiger und ein quadratischer Querschnitt, aber auch unregelmäßige Verteilungen der Querschnitte möglich.

Die Form des Querschnitts der Nut hat dabei wiederum Einfluss auf die Flexibilität bzw. das Elastizitätsmodul des Stabes und kann somit dafür verwendet werden, die Bewegung des zu korrigierenden Gelenks in bestimmte Richtungen zu verhindern bzw. beschränkt zu ermöglichen und somit die Orthese oder Prothese derart anzupassen, dass an die spezifische Fehlstellung angepasst ist.

Bevorzugterweise weist der mindestens eine Stab des Verbindungselements auf oder besteht aus Carbonfaser, Glasfaser, Aluminium, Titan, Stahl, Kunststoff, Keramik, Holz oder eine Kombination dieser Materialien. Das für den Stab eingesetzt Material dient als Grundlage für die Flexibilität der Orthese oder Prothese. So hat beispielsweise Keramik einen höheren Elastizitätsmodul als Titan oder Aluminium und ein Stab aus Keramik ist somit steifer, weist also eine geringere Flexibilität auf, als ein gleicher Stab aus Titan oder Aluminium.

Ausgehend von dem Elastizitätsmodul des gewählten Materials bzw. der gewählten Materialkombination kann die Orthese oder Prothese und die damit verbunden Bewegungsfreiheit des zu korrigierenden Gelenks mit den Nuten und deren Eigenschaften angepasst werden.

Bevorzugterweise kann der Stab ein Schutzelement, beispielsweise eine Schutzhülle oder ein Stabiliesierungselement aufweisen, das ein zu starkes Durchbiegen des Stabes der Orthese oder der Prothese erfolgt oder dass dieser Stab bricht. Selbst wenn der Stab bricht, kann die Verletzungsgefahr verringert werden, da durch die Schutzverkleidung Bruchstücke weiterhin zusammengehalten werden. Zugleich soll das Schutzelement so ausgestaltet sein, dass die Bewegung der Orthese oder Prothese funktionell nicht eingeschränkt wird. Die Schutzverkleidung kann dabei als Gewebeschlauch oder als 3D-Druckteil ausgestaltet sein.

Bevorzugterweise ist das 3D-Druckteil dabei derart ausgestaltet, dass es den Stab umschließt, wobei die Seite, die sich entlang der Längserstreckungsrichtung einer der Hauptoberflächen des Stabes erstreckt als durchgängige Fläche ausgebildet ist.

Ist das Schutzelement als Stab ausgebildet, kann der Stab Rippen aufweisen, die zu einer Seite des Stabes hervorstehen und in Längsrichtung des Stabes hineinander angeordnet sind. Bevorzugterweise kann der Abstand der Rippen zueinander gleich dem Abstand der vorgenannten Nuten zueinander sein, so dass die Rippen in die Nuten eingreifen können

Bevorzugterweise weist der Stab des Schutzelementes an den in Längserstreckungsrichtung des Stabes liegenden Enden Schlaufen aus, die ihrerseits an den Befestigungsvorrichtungen der Orthese oder Prothese angebracht werden können.

Bevorzugterweise kann das Schutzelement aus Kunststoff bestehen. Die Wahl des Materials ist dabei von dem gewählten Material für den Stab und dessen Elastizitätsmodul abhängig. Weiterhin kann die Wahl unter Berücksichtigung der für ein additives Fertigungsverfahren (3D-Druck) zur Verfügung stehenden Materialien erfolgen. Da die Bewegungsfreiheit des Stabes durch die Schutzverkleidung, in diesem Fall durch das 3D-Druckteil, nicht vollständig beeinträchtigt werden soll, muss das Material derart gewählt werden, dass dies gewährleistet werden kann.

Die Nuten sind erfindungsgemäß derart angeordnet, dass der Stab bei Auslenkung seiner beiden Enden im Wesentlichen senkrecht zu seinen Hauptoberflächen für beide Auslenkungsrichtungen unterschiedliche Elastizitätsmodule aufweist.

Vorteilhafterweise weist der zumindest eine Stab in einer Ebene senkrecht zur Längserstreckung des Stabs eine im Wesentlichen rechteckige, quadratische, halbrunde, elliptische, trapezförmige, dreieckige, vieleckige, rautenförmige, konvexe oder konkave Form hat oder entlang der Längsrichtung des Stabes eine konische Form mit runder oder elliptischer Grundfläche auf.

Vorteilhafterweise ist das Schutzelement ein Gewebeschlauch, der den Stab zumindest bereichsweise umgibt.

Im Gegensatz zum in der Einleitung beschriebenen Stand der Technik zur Behandlung eines Klumpfußes, lässt die Fußeinheit verbunden mit einer Unterschenkeleinheit durch einen erfindungsgemäßen dynamischen Stabilitätsträger eine physiologische Pronation im Subtalargelenk bei Fersenauftritt zu.

So kann beispielsweise die Tibia (das Schienbein) eine Innenrotation durchführen, wodurch ein gedämpfter Fersenauftritt beim Erstkontakt bzw. bei der Ladeantwort (Belastungsantwort) möglich ist. Durch die zahlreichen Einstellungsmöglichkeiten bei der Konstruktion des dynamischen Stabilitätsträgers können die Rotationskräfte beim Fersenauftritt derart reduziert werden, dass sich für den Patienten ein weiches, federndes Moment ergibt. Dabei kann die Härte des dynamischen Stabilitätsträgers bzw. die Amplitude der Rotation an die Bedürfnisse des Patienten bzw. des Befundes angepasst werden. Beim Verlassen der Standbeinphase wird das Subtalargelenk durch die Orthese oder Prothese wieder kontrolliert in die Neutralstellung zurückgeführt wodurch die Eversion reduziert wird. Dadurch gewährt eine erfindungsgemäße Orthese oder Prothese eine definierte Mobilität des Fußgelenks in Bezug auf die physiologische Bewegung bei gleichzeitiger bestehender und nötiger Stabilität in Bezug auf die pathologische Bewegung. Der Rück- und Mittelfuß werden durch die gleichbleibende Konstruktion der Prothese oder Orthese wie in standardgemäßen Prothesen oder Orthesen gehalten.

Es zeigen:
- Fig. 1:: ein Bein mit Orthese oder Prothese in Seitenansicht;
- Fig. 2:: einen Stab eines Verbindungselements mit mehreren Lagen;
- Fig. 3:: einen Unterschenkel mit Orthese oder Prothese in Frontalansicht;
- Fig. 4:: den flachen Stab mit orthogonalen Nuten in Biegung mit Vergrößerungen;
- Fig. 5:: den flachen Stab mit orthogonalen der Fig. 4 und einen flachen Stab mit schrägen Nuten in Biegung und Torsion;
- Fig. 6:: die Stäbe der Fig. 5 in kombinierter Biegung und Torsion und
- Fig. 7:: eine Tabelle mit beispielhaften Parametern für den flachen Stab in Bezug auf das Gewicht eines Patienten;
- Fig. 8:: Schutzverkleidung und Orthese oder Prothese mit montierter Schutzverkleidung.

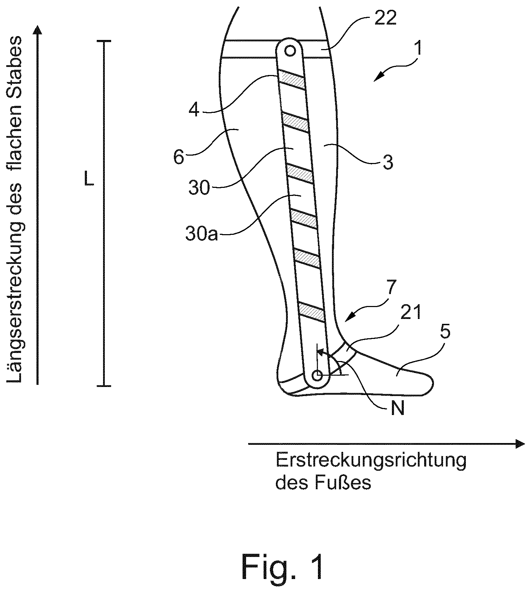

Es ist zu beachten, dass die Nuten in den Fig. 1-3 nicht skalengerecht dargestellt sind, sondern deutlich vergrößert wurden, um die verschiedenen Parameter besser erkennbar zu machen.

Fig. 1 zeigt eine Orthese bzw. Prothese 1, die an einem Bein befestigt ist. Die Orthese bzw. Prothese weist eine erste Befestigungsvorrichtung 21, die an einem Fuß 5 befestigt ist, und eine zweite Befestigungsvorrichtung 22, die an einem Unterschenkel 6 desselben Beins befestigt ist. Damit ist das zu behandelnde Gelenk 7 das Fußgelenk. Die beiden Befestigungsvorrichtungen 21, 22 sind mittels eines Verbindungselements 3, das als ein flacher Stab 30 ausgebildet ist, verbunden. Der flache Stab 30 weist eine erste Hauptoberfläche 30a auf. In der ersten Hauptoberfläche 30a des flachen Stabes 30 sind Nuten 4 eingelassen, die einen im Wesentlichen rechteckigen Querschnitt haben und geradlinig verlaufen. Der Winkel W, den die Nuten 4 mit einer Richtung entlang einer Längserstreckung des flachen Stabes 30 einschließen beträgt weniger als 90°. Die Nuten können auch senkrecht auf die Richtung entlang der Längserstreckung des flachen Stabes 30 stehen. Ein Neigungswinkel N ist definiert als der Winkel von den beiden Hauptoberflächen 30a, 30b und der Ebene senkrecht zur Erstreckungsrichtung des ersten Gliedes, bei einer Rotation um eine Rotationsachse der Flexion des zu behandelnden Gelenks eingeschlossen wird. In der Fig. 1 ist der die Orthese bzw. Prothese derart ausgerichtet, dass der Neigungswinkel N 90° beträgt. Eine Länge L des flachen Stabes 30 ist definiert als die Erstreckung des flachen Stabes entlang der Längserstreckung von der ersten Befestigungsvorrichtung 21 zur zweiten Befestigungsvorrichtung 22 definiert. Die Länge ist abhängig von der Art des zu korrigierenden Gelenks 7, sowie der Größe des Patienten.

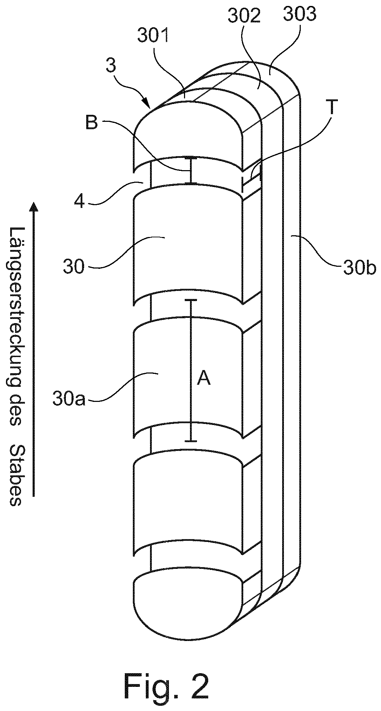

Fig. 2 zeigt einen flachen Stab 30 des Verbindungselements in schräger Ansicht. Der flache Stab 30 weist in einer Ebene senkrecht zu einer Achse entlang der Längserstreckung des flachen Stabes 30 einen rechteckigen Querschnitt auf. Aus einer Richtung senkrecht zur ersten Hauptoberfläche 30a des flachen Stabes 30 weisen die Enden des ersten flachen Stabes 31 eine elliptische Form auf. Der flache Stab 30 besteht aus drei unterschiedlichen Lagen 301, 302, 303, wobei eine Seite der ersten Lage 301 als erste Hauptoberfläche 30a des flachen Stabes 30 und eine Seite der dritten Lage 303 als zweite Hauptoberfläche 30b des flachen Stabes 30 dient. Die zweite Lage 302 des flachen Stabes 30 ist zwischen den beiden anderen Lagen 301, 303 eingebettet. In der ersten Hauptoberfläche 30a sind weiterhin vier Nuten 4 eingebracht, die eine gekrümmte Form aufweisen. Die Tiefe T der Nuten ist dabei in die Richtung definiert, die senkrecht auf die, die Nuten enthaltende Hauptoberfläche 30a steht und von der einen, die Nut 4 enthaltenden, ersten Hauptoberfläche 30a in Richtung hin zur anderen, zweiten Hauptoberfläche 30b verläuft. Die Tiefe T der Nuten 4 kann sich beispielsweise, wie in Fig. 2, über die Dicke der gesamten ersten Lage 301 erstrecken. Die Breite B der Nut 4 ist, bei geradlinigem Verlauf der Nut 4 als diejenige Erstreckung in eine Richtung parallel zur die Nut 4 aufweisenden ersten Hauptoberfläche 30a und senkrecht zur Ausbreitungsrichtung der Nut 4 anzusehen. Im Fall einer gekrümmten Nut 4 ist die Breite B der Nut 4 als diejenige Erstreckung einer Richtung parallel zur ersten Hauptoberfläche 30a und senkrecht zur örtlichen Verlaufsrichtung der Nut 4 definiert. Der Abstand A der Nuten 4 dabei in einer Richtung parallel zur Längserstreckung des flachen Stabes 30 definiert und wird vom Mittelpunkt der einer Nut 4 zum Mittelpunkt der nächsten Nut 4 gemessen.

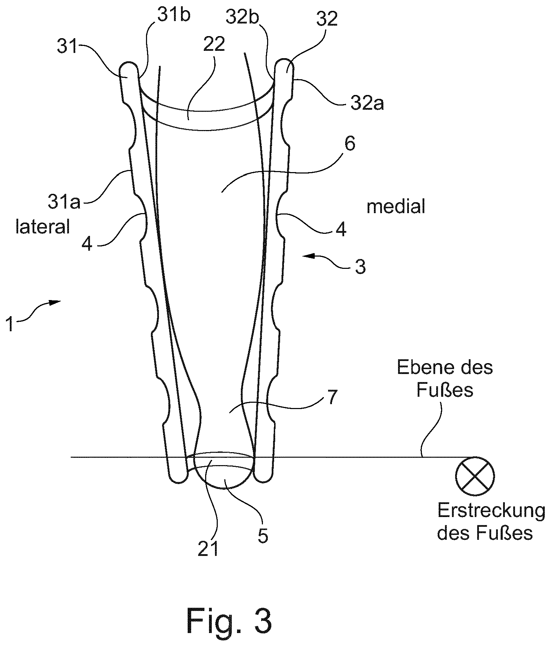

Fig. 3 zeigt die Orthese bzw. Prothese 1 in der Rückansicht des Beines. Die Orthese bzw. Prothese 1 ist mit der ersten Befestigungsvorrichtung 21 am Fuß 5 und mit der zweiten Befestigungsvorrichtung 22 am Unterschenkel 6 des Beins angebracht. Das zu korrigierende Gelenk 7 ist somit wieder das Fußgelenk. Das Verbindungselement 3, das die beiden Befestigungsvorrichtungen 21, 22 miteinander verbindet weist einen ersten flachen Stab 31 und einen zweiten flachen Stab 32 auf. Beide Stäbe haben jeweils eine erste Hauptoberfläche 31a, 32a, die jeweils in entgegengesetzte Richtungen und in eine Richtung weg vom Unterschenkel 6 ausgerichtet sind. Die beiden zweiten Hauptoberflächen 31b, 32b dagegen sind einander zugewandt und zeigen jeweils in Richtung des Unterschenkels 6. Beide flachen Stäbe 31, 32 weisen jeweils in ihrer ersten Hauptoberfläche 31a, 32a vier Nuten 4 auf, die einen elliptischen Querschnitt in einer Ebene senkrecht zur jeweiligen ersten Hauptoberfläche 31a, 32a haben. Die Breite B der Nuten 4 ist nicht für alle vier der Nuten 4 gleich, sondern ändert sich in der Richtung entlang der Längserstreckung des jeweiligen Stabes 31, 32.

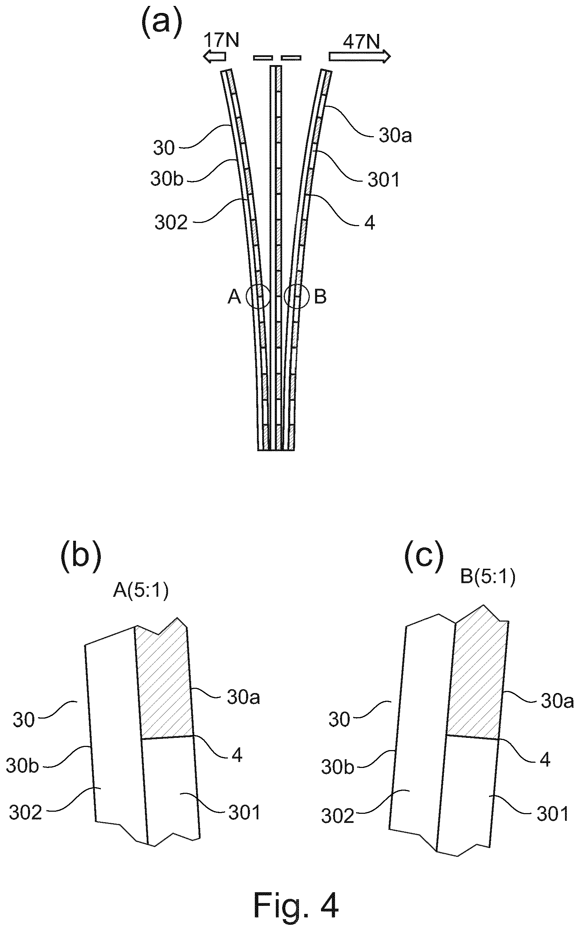

Fig. 4 zeigt einen flachen Stab 30 in drei verschiedenen Positionen. In der ersten (mittleren) Position befindet sich der flache Stab 30 in einer neutralen Position, in der keine Kräfte auf ihn ausgeübt werden. Der flache Stab 30 besteht aus zwei Lagen 301, 302 und weist eine erste Hauptoberfläche 30a auf, die sich auf der nach außen gerichteten Seite der ersten Lage 301 befindet. Eine zweite Hauptoberfläche 30b auf des flachen Stabes 30 befindet sich auf der nach außen gerichteten Seite der zweiten Lage 302. Die vierzehn Nuten 4 des flachen Stabes 30, die sich jeweils an den Farbübergängen (schwarz zu grau bzw. grau zu schwarz) befinden, sind in der ersten Lage 301 eingelassen. Die Nuten 4 haben einen im Wesentlichen rechteckigen Querschnitt und sind derart angeordnet, dass die Nuten 4 sich in eine Richtung senkrecht zur Längserstreckung des flachen Stabes 30 erstrecken. In der linken Abbildung des flachen Stabes 30 der Fig. 4 (a) wird der flache Stab 30 in einer ausgelenkten Position dargestellt. Die Auslenkung ist dabei in eine Richtung senkrecht zur ersten und zweiten Hauptoberfläch 30a, 30b des flachen Stabes 30 und in einer Richtung von der ersten Hauptoberfläche 30a zur zweiten Hauptoberfläche 30b gerichtet. Bei einer Auslenkung in diese Richtung werden die Nuten 4 auseinandergezogen. Der markierte Bereich A ist vergrößert in Fig. 4 (b) gezeigt. Der Spalt der durch die Nut gebildet wird bei Belastung in die beschriebene Richtung gestreckt und vergrößert sich. Auf der rechten Abbildung der Fig. 4 (a) ist der Stab in einer Position gezeigt, in dem die Auslenkung in die entgegengesetzte Richtung des zuvor beschriebenen Beispiels durchgeführt wird, also in Richtung von der zweiten Hauptoberfläche 30b zur ersten Hauptoberfläche 30a. Bei einer Auslenkung in diese Richtung werden die Nuten 4 des flachen Stabes 30 zusammengedrückt. Der Ausschnitt B ist vergrößert in Fig. 4 (c) zu sehen. Der Spalt, der durch die Nut 4 gebildet wird, wird komplett zusammengedrückt. Bei weiterer Auslenkung ist das Verhalten des flachen Stabes 30 ähnlich zu dem eines flachen Stabes 30 ohne Nuten 4. Bei einer Auslenkung des flachen Stabes 30 mit gleicher Amplitude aber in die beschriebenen entgegengesetzten Richtungen müssen unterschiedliche Kräfte aufgewendet werden. Im Fall der Auslenkung des ersten Beispiels ist eine Kraft von 17 N notwendig, während im zweiten Beispiel eine Kraft von 47 N notwendig ist. Die eingebrachten Nuten 4 beeinflussen bei einer Auslenkung senkrecht zu den beiden Hauptoberflächen 30a, 30b abhängig von der Richtung den Elastizitätsmodul in unterschiedlicher Weise und daher ist die Bewegung in eine Richtung stärker beschränkt als in die entgegengesetzte.

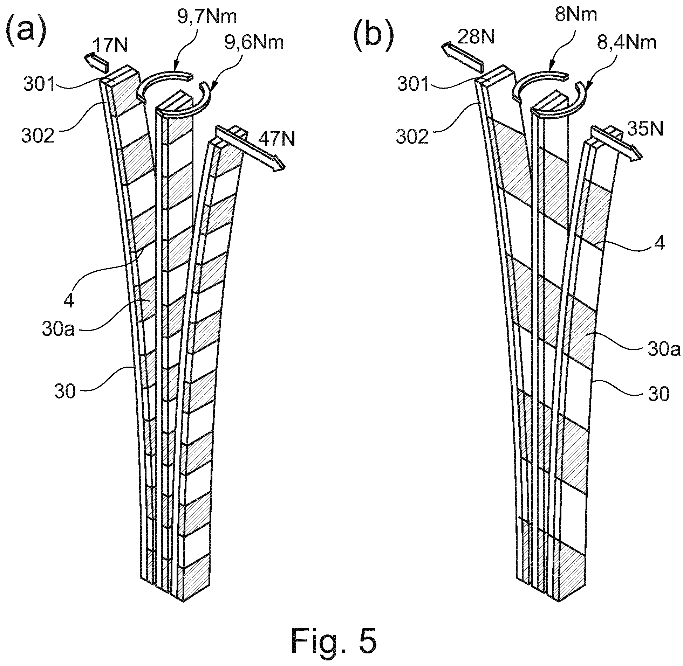

Fig. 5 (a) zeigt den flachen Stab 30 der Fig. 4 (a), wobei gleiche Bezugszeichen gleiche Merkmale markieren. Zusätzlich zur Auslenkung des flachen Stabes 30 in Richtungen senkrecht zu den beiden Hauptoberflächen 30a, 30b wird auch die Kraft gezeigt, die für eine Torsion durch Rotation um eine Achse, die entlang der Längserstreckung des flachen Stabes 30 und durch einen Mittelpunkt des flachen Stabes 30 verläuft, notwendig ist. Die Kräfte für eine Torsion in beide Richtungen im (9,6 Nm) und gegen (9,7 Nm) den Uhrzeigersinn sind bei gleicher Auslenkung nahezu identisch. Das lässt sich darauf zurückführen, dass die Nuten 4 derart angeordnet sind, dass sie sich in eine Richtung senkrecht zur Längserstreckung des flachen Stabes 30 erstrecken und dadurch bei der Torsion in beide Richtungen gleich verhalten. Fig. 5 (b) zeigt einen flachen Stab 30 ähnlich zu dem der Figuren 4 (a) und 5 (a). Gleiche Bezugszeichen bezeichnen wieder gleiche Merkmale und werden nicht näher erläutert. Die Unterschiede zu den beiden genannten Figuren besteht darin, dass zum einen sieben Nuten 4 über den flachen Stab 30 verteilt sind (anstelle von vierzehn) und diese nicht senkrecht zur Längserstreckung des Stabes 30 verlaufen, sondern mit ihr einen Winkel W von 45° einschließen. Auslenkungen werden wie bereits zur Fig. 4 (a) beschrieben durchgeführt. Dabei fällt auf, dass wiederum für die Auslenkung in eine Richtung, in der die Nuten 4 zusammengedrückt werden, mehr Kraft (35 N) notwendig ist als für eine Auslenkung in eine Richtung, in der die Nuten 4 auseinandergezogen werden (28 N). Der Unterschied des Kraftaufwands ist allerdings wesentlichen geringer, als in dem Beispiel der Nuten 4, die senkrecht zur Längserstreckung des flachen Stabes 30 verlaufen (Fig. 4 (a) und Fig. 5 (a)). Im Gegensatz zur Torsion die im Beispiel der Fig. 5 (a) gezeigt ist, hat der flache Stab 30 aufgrund des nicht orthogonalen Winkels W in der Fig. 5 (b) eine Vorzugsrichtung. Für eine Torsion durch Rotation im Uhrzeigersinn ist ein Drehmoment von 8,4 Nm nötig, während für eine Torsion durch Rotation mit gleicher Auslenkung gegen den Uhrzeigersinn nur ein Drehmoment von 8,0 Nm notwendig ist. Der Grund dafür ist, dass im Fall der Torsion im Uhrzeigersinn die Nuten 4 zusammengedrückt werden und sich ab einem bestimmten Zeitpunkt ähnlich zu einem flachen Stab 30 ohne Nuten 4 verhalten, während im Fall der Torsion gegen den Uhrzeigersinn die Nuten 4 auseinandergezogen werden und somit weniger Widerstand vorhanden ist.

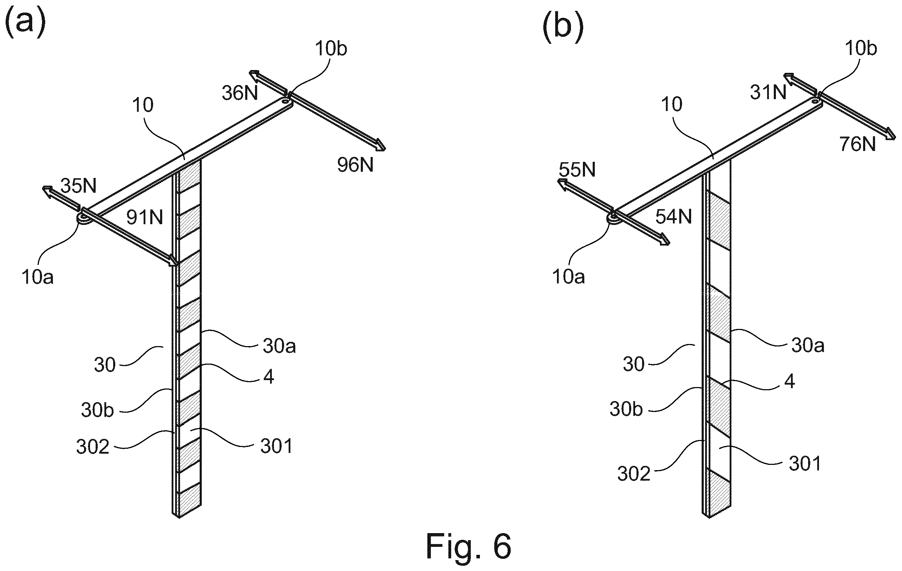

Fig. 6 (a) und Fig. 6 (b) zeigen jeweils dieselben flachen Stäbe 30 wie Fig. 5 (a) und Fig. 5 (b). Gleiche Bezugszeichen beschreiben in der Fig. 6 die gleichen Merkmale wie in Fig. 5 und werden daher nicht erneut erläutert. Im Gegensatz zu den zuvor gezeigten Beispielen ist an einem Ende des flachen Stabes 30 eine Platte 10 angebracht, deren Längserstreckung parallel zu den beiden Hauptoberflächen 30a, 30b des flachen Stabes 30 verläuft. Die Platte 10 weist an beiden Enden 10a, 10b Löcher auf, mit denen die Platte 10 und somit der flache Stab 30 fixiert werden kann. Zur Messung der notwendigen Kraft einer kombinierten Torsion und Biegung wird das erste Ende 10a der Platte 10 fixiert und die Kraft am zweiten Ende 10b aufgewandt. Die Richtung in die die Kraft aufgewandt wird ist dieselbe, die bereits zur Biegung in den vorherigen Beispielen beschrieben wurde. Da allerdings der Angriffspunkt der Kraft nicht direkt am flachen Stab 30, sondern seitlich in einer Richtung parallel zu den Hauptoberflächen 30a, 30b des flachen Stabes 30 versetzt liegt, ruft eine Kraft in diese Richtung nicht nur eine Biegung, sonder auch eine Torsion hervor. Nachdem die Messungen in beide Richtungen durchgeführt worden sind, wird das zweite Ende 10b fixiert und die Kraft am ersten Ende 10a aufgebracht. In Fig. 6 (a) ist das Ergebnis der Messungen an einem flachen Stab 30 mit senkrecht zur Längserstreckung des flachen Stabes 30 verlaufenden Nuten 4 zu sehen. Da die Kraft, die für eine Torsion aufgebracht werden muss, in beiden Rotationsrichtungen im und gegen den Uhrzeigersinn annähernd gleich ist (vgl. Fig. 5 (a)), ist auch im Fall der kombinierten Torsion und Biegung das Ergebnis in den Fällen, in denen die Kraft in die gleiche Richtung aufgewandt ist annähernd gleich (35 N und 36 N bzw. 91 N und 96 N). Der Unterschied der aufzuwendenden Kraft um dieselbe Auslenkung zu erreichen kommt aus den Ergebnissen der Biegung (vgl. Fig. 5 (a)), da eine Biegung die die Nuten 4 des flachen Stabes 30 staucht mehr Kraft benötigt, als eine Biegung die die Nuten 4 des flachen Stabes 30 streckt. Ein anderes Ergebnis ist für den flachen Stab 30 der Fig. 6 (b) zu sehen, deren Nuten 4 die mit der Längserstreckung des flachen Stabes 30 einen Winkel W von 45° einschließen. Da die schrägen Nuten 4 eine Vorzugsrichtung in Bezug auf die Torsion haben, unterscheiden sich die aufzuwenden Kräfte auch bei Kraftwirkung in die gleiche Richtung. Da eine Torsion durch Rotation im Uhrzeigersinn mehr Kraft benötigt als eine Torsion durch Rotation gegen den Uhrzeigersinn unterscheiden sich auch die Kräfte die nötig sind dieselbe Auslenkung zu erzeugen. Eine Kraft, die am ersten Ende 10a der Platte 10 in Richtung von der zweiten Hauptoberfläche 30b zur ersten Hauptoberfläche 30a aufgewandt wird (54 N), ist kleiner als eine Kraft in dieselbe Richtung, die aber am zweiten Ende 10b der Platte 10 aufgewandt wird (76 N). Selbiges Verhalten ist auch bei Aufwendung der Kraft in die entgegengesetzte Richtung zu sehen, wobei die Kraft die aufgewendet werden muss am ersten Ende 10a der Platte 10 (55 N) größer ist als die am zweiten Ende 10b der Platte 10 aufgewandte Kraft (31 N). Insgesamt ist die Kraft im zweiten Fall wieder niedriger, da die Biegung dieses Falls die Nuten 4 streckt (vgl. Fig. 5 (b)).

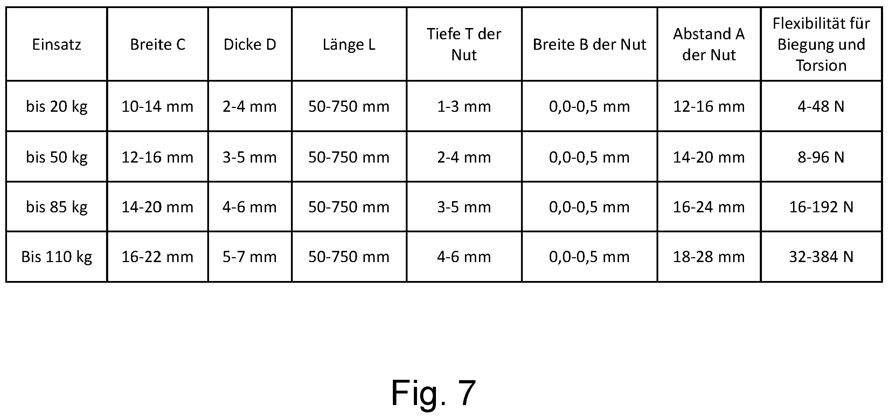

Fig. 7 zeigt eine Tabelle mit Beispielen für Werte zur Konstruktion eines erfindungsgemäßen dynamischen Stabilitätsträgers. In der ersten Spalte der Tabelle ist das Gewicht des zu behandelnden Patienten angegeben. Ausgehend von diesem Gewicht, zeigt die Tabelle einen Wertebereich für die Breite C, Dicke D und Länge L des flachen Stabes 30 oder der flachen Stäbe 31, 32, sowie Parameter für die Tiefe T, Breite B und Abstand A der Nuten 4. In der letzten Spalte sind Wertebereiche für die Flexibilität der Biegung und Torsion angegeben, die mit den zuvor angegebenen Parametern erreicht werden können. So sind beispielsweise für einen erwachsenen Mann mit einem Gewicht von 80 kg Werte der fünften Zeile zu verwenden. Der Stab sollte dabei eine Breite zwischen 14 mm und 20 mm, eine Dicke zwischen 3 mm und 5 mm und eine Länge zwischen 50 mm und 750 mm, wobei die Länge vom behandelten Gelenk und der Größe des Patienten abhängt. Die Nuten haben dabei eine Tiefe T von 3 mm bis 5 mm, eine Breite B kleiner oder gleich 0,5 mm und einen Abstand A zueinander zwischen 16 mm und 24 mm. Diese Werte ermöglichen eine Flexibilität des dynamischen Stabilitätsträgers bei Biegung und Torsion von 10N bis 300N, vorteilhafterweise von 16 N bis zu 192 N.

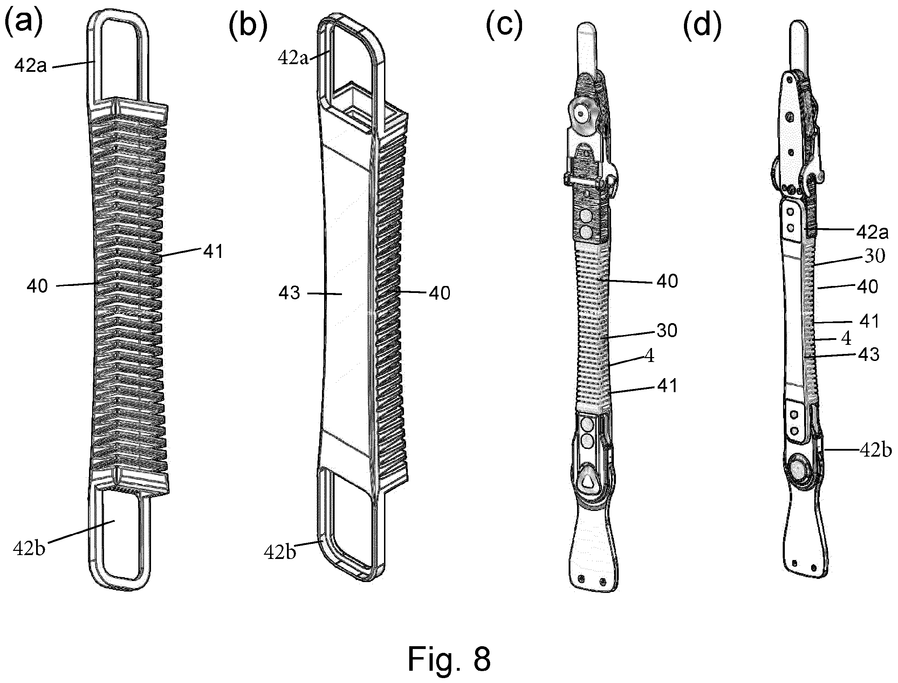

Fig. 8 (a) und 8 (b) zeigen ein 3D-Druckteil 40, das als Schutzelement für den flachen Stab 30 einer erfindungsgemäßen Prothese oder Orthese (hier in Fig. 8a und 8b nicht dargestellt)dient, jeweils in Frontalansicht (Fig. 8a) und Rückansicht (Fig. 8b). Das Schutzelement weist in Form eines ebenfalls flachen Stabes 43 eine längliche Erstreckung auf, die derjenigen des durch es geschützten Stabes 30 im Wesentlichen entspricht. In Länserstreckung des Schutzelementes 40 sind eine Vielzahl von Rippen 41 benachbart zueinander angeordnet. Zwischen den einzelnen Rippen 41 ist jeweils ein freier Abstand vorgesehen. Der Abstand der Rippen 41 zueinander kann dabei frei gewählt werden, sodass die Orthese oder Prothese durch das 3D-Druckteil 40 nicht an einer Durchbiegung gehindert wird. Die elastischen Eigenschaften des Stabes 30 der Orthese werden also nicht oder kaum beeinträchtigt wird. Allerdings bilden bei zu großer Durchbiegung die Rippen 41 einen Anschlag und verhindern so eine zu große Durchbiegung des Stabes 30 oder gar ein Brechen des Stabes 30. In einem bevorzugten Fall können die Abstände der Rippen 41 zueinander derart gewählt sein, dass sie mit dem Abstand A der vorerwähnten Nuten 4 der erfindungsgemäßen Prothese oder Orthese übereinstimmen.

Das Schutzelement 40 weist an seinen beiden Längsenden Schlaufen 42 auf. Diese dienen dazu, die vorerwähnte ste und zweite Befestigungsvorrichtung 21, 22 der Orthese oder Prothese an dem 3D-Druckteil 40 zu befestigen.

Fig. 8 (c) und 8 (d) zeigen eine erfindungsgemäße Orthese oder Prothese, die mit dem Schutzelement 40 ausgestattet ist, in Ansichten von zwei gegenüberliegenden Seiten. Die Rippen 41 greifen in die Nuten 4 des Stabes 30 der Prothese oder Orthese ein. Das Schutzelement kann mit dem Stab 30 der Orthese beispielsweise durch Verschrauben, Vernieten oder Verkleben verbunden sein.

## Patentansprüche

1. Orthese oder Prothese (1) zur Korrektur einer Fehlstellung eines zwei Glieder verbindenden Gelenks, insbesondere eines Fußgelenks, mit einer ersten Befestigungsvorrichtung (21) und einer zweiten Befestigungsvorrichtung (22), wobei die beiden Befestigungsvorrichtungen (21, 22) jeweils an einem ersten und einem zweiten der beiden Glieder befestigbar sind,
einem Verbindungselement (3), das die beiden Befestigungsvorrichtungen (21, 22) miteinander verbindet,
wobei das Verbindungselement (3) mindestens einen flachen Stab (30, 31, 32) mit zwei im Wesentlichen parallel zueinander verlaufenden Hauptoberflächen (30a, 30b, 31a, 31b, 32a, 32b) aufweist, der auf mindestens einer oder genau einer seiner Hauptoberflächen (30a, 30b, 31a, 31b, 32a, 32b) eine Vielzahl von in der Längserstreckung des Stabes (30, 31, 32) von der ersten Befestigungsvorrichtung (21) zur zweiten Befestigungsvorrichtung (22) voneinander beabstandet angeordnete Nuten (4) aufweist, wobei die Nuten (4) sich zumindest teilweise nicht vollständig sondern bereichsweise über die quer zur Längserstreckungsrichtung verlaufende Breite des Stabes (30, 31, 32) erstrecken und unter einem Winkel W mit 10 °≤ |W| ≤ 90 ° zur Längserstreckung des Stabes (30, 31, 32) verlaufen, **dadurch gekennzeichnet, dass** die Nuten auf der einen oder den beiden Hauptoberflächen (30a, 30b, 31a, 31b, 32a, 32b) derart angeordnet sind, dass eine Auslenkung in eine der beiden Richtungen senkrecht zu den beiden im Wesentlichen parallelen Hauptoberflächen (30a, 30b, 31a, 31b, 32a, 32b) einen anderen Elastizitätsmodul aufweist als eine Auslenkung in die entgegengesetzte Richtung.

2. Orthese oder Prothese (1) nach dem vorhergehenden Anspruch,
**dadurch gekennzeichnet, dass** der flache Stab (30, 31, 32) eine, zwei oder mehrere Lagen (301, 302, 303) aufweist, die sich im Wesentlichen parallel zu mindestens einer der Hauptoberflächen (30a, 30b, 31a, 31b, 32a, 32b) des mindestens einen Stabes (30, 31, 32) erstrecken.

3. Orthese oder Prothese (1) nach einem der vorhergehenden Ansprüche, **dadurch gekennzeichnet, dass** der flache Stab (30, 31, 32) eine Breite C mit 5 mm ≤ C ≤ 35 mm, vorteilhafterweise 7 mm ≤ C ≤ 25 mm, vorteilhafterweise 10 mm ≤ C ≤ 22 mm aufweist.

4. Orthese oder Prothese (1) nach einem der vorhergehenden Ansprüche, **dadurch gekennzeichnet, dass** der flache Stab (30, 31, 32) eine Dicke D mit 0,5 mm ≤ D ≤ 15 mm, vorteilhafterweise 1 mm ≤ D ≤ 10 mm, vorteilhafterweise 2 mm ≤ D ≤ 7 mm aufweist.

5. Orthese oder Prothese (1) nach einem der vorhergehenden Ansprüche, **dadurch gekennzeichnet, dass** der zumindest eine flache Stab (30, 31, 32) eine Länge L mit 20 mm ≤ L ≤ 1000 mm, vorteilhafterweise 30 mm ≤ L ≤ 900 mm, vorteilhafterweise 50 mm ≤ L ≤ 750 mm aufweist.

6. Orthese oder Prothese (1) nach einem der vorhergehenden Ansprüche, **dadurch gekennzeichnet, dass** die Nuten (4) unter einem Winkel W mit 30 ° ≤ |W| ≤ 90°, insbesondere senkrecht zur Längserstreckung verlaufen.

7. Orthese oder Prothese (1) nach einem der vorhergehenden Ansprüche, **dadurch gekennzeichnet, dass** die Nuten (4) zumindest abschnittsweise geradlinig und/oder zumindest abschnittsweise gekrümmt verlaufen.

8. Orthese oder Prothese (1) nach einem der vorhergehenden Ansprüche, **dadurch gekennzeichnet, dass** mindestens eine, mehrere oder alle der Nuten (4) eine Tiefe T mit 0,3 mm ≤ T ≤ 10 mm, vorteilhafterweise 1 mm ≤ T ≤ 6 mm, vorteilhafterweise 2 mm ≤ T ≤ 5 mm aufweist.

9. Orthese oder Prothese (1) nach einem der vorhergehenden Ansprüche, **dadurch gekennzeichnet, dass** mindestens eine, mehrere oder alle Nuten (4) eine Breite B mit 0,05 mm ≤ B ≤ 10 mm, vorteilhafterweise 0,1 mm ≤ B ≤ 5 mm, vorteilhafterweise 0,1 mm ≤ B ≤ 0,5 mm aufweisen.

10. Orthese oder Prothese (1) nach einem der vorhergehenden Ansprüche, **dadurch gekennzeichnet, dass** mindestens eine, mehrere oder alle Nuten (4) zueinander in Richtung der Längserstreckung des Stabes (30, 31, 32) einen Abstand A mit 5 mm ≤ A ≤ 40 mm, vorteilhafterweise 10 mm ≤ A ≤ 35 mm, vorteilhafterweise 12 mm ≤ A ≤ 28 mm aufweist.

11. Orthese oder Prothese (1) nach einem der vorhergehenden Ansprüche, **dadurch gekennzeichnet, dass** die erste Befestigungsvorrichtung (21) mit einem Fuß (5) einer Person und die zweite Befestigungsvorrichtung (22) mit einem Unterschenkel (6) der Person verbindbar sind.

## Claims

1. Orthosis or prosthesis (1) for correcting a malposition of a joint connecting two limbs, in particular an ankle joint, with a first fastening device (21) and a second fastening device (22), wherein the two fastening devices (21, 22) can each be fastened to a first and a second of the two limbs,
a connecting element (3) which connects the two fastening devices (21, 22) to one another,
wherein the connecting element (3) comprises at least one flat bar (30, 31, 32) with two main surfaces (30a, 30b, 31a, 31b, 32a, 32b) extending substantially parallel to each other, which bar (30, 31, 32) has on at least one or exactly one of its main surfaces (30a, 30b, 31a, 31b, 32a, 32b) a plurality of grooves (4) arranged spaced apart from each other in the longitudinal extension of the bar (30, 31, 32) from the first fastening device (21) to the second fastening device (22), wherein the grooves (4) extend at least in parts not completely but in regions over the width of the bar (30, 31, 32), which extends transversal to the longitudinal extension of the bar, and extend at an angle W of 10 °≤ | W | ≤ 90 ° to the longitudinal extension of the bar (30, 31, 32), **characterized in that** the grooves on the one or on both of the main surfaces (30a, 30b, 31a, 31b, 32a, 32b) are arranged in such a way that a deflection in one of the two directions perpendicular to the two essentially parallel main surfaces (30a, 30b, 31a, 31b, 32a, 32b) has a different modulus of elasticity than a deflection in the opposite direction.

2. Orthosis or prosthesis (1) according to the preceding claim, **characterized in that** the flat bar (30, 31, 32) comprises one, two or more layers (301, 302, 303) extending substantially parallel to at least one of the main surfaces (30a, 30b, 31a, 31b, 32a, 32b) of the at least one bar (30, 31, 32).

3. Orthosis or prosthesis (1) according to one of the preceding claims, **characterized in that** the flat bar (30, 31, 32) has a width C of 5 mm ≤ C ≤ 35 mm, advantageously 7 mm ≤ C ≤ 25 mm, advantageously 10 mm ≤ C ≤ 22 mm.

4. Orthosis or prosthesis (1) according to one of the preceding claims, **characterized in that** the flat bar (30, 31, 32) has a thickness D of 0.5 mm ≤ D ≤ 15 mm, advantageously 1 mm ≤ D ≤ 10 mm, advantageously 2 mm ≤ D ≤ 7 mm.

5. Orthosis or prosthesis (1) according to one of the preceding claims, **characterized in that** the at least one flat bar (30, 31, 32) has a length L of 20 mm ≤ L ≤ 1000 mm, advantageously 30 mm ≤ L ≤ 900 mm, advantageously 50 mm ≤ L ≤ 750 mm.

6. Orthosis or prosthesis (1) according to one of the preceding claims, **characterized in that** the grooves (4) extend at an angle W of 30° ≤ | W | ≤ 90 °, in particular perpendicular to the longitudinal extension.

7. Orthosis or prosthesis (1) according to one of the preceding claims, **characterized in that** the grooves (4) run in a straight line at least in sections and/or are curved at least in sections.

8. Orthosis or prosthesis (1) according to one of the preceding claims, **characterized in that** at least one, several or all of the grooves (4) have a depth T of 0.3 mm ≤ T ≤ 10 mm, advantageously 1 mm ≤ T ≤ 6 mm, advantageously 2 mm ≤ T ≤ 5 mm.

9. Orthosis or prosthesis (1) according to one of the preceding claims, **characterized in that** at least one, several or all grooves (4) have a width B of 0.05 mm ≤ B ≤ 10 mm, advantageously 0.1 mm ≤ B ≤ 5 mm, advantageously 0.1 mm ≤ B ≤ 0.5 mm.

10. Orthosis or prosthesis (1) according to one of the preceding claims, **characterized in that** at least one, several or all grooves (4) have a distance A of 5 mm ≤ A ≤ 40 mm, advantageously 10 mm ≤ A ≤ 35 mm, advantageously 12 mm ≤ A ≤ 28 mm from one another in the direction of the longitudinal extension of the bar (30, 31, 32).

11. Orthosis or prosthesis (1) according to one of the preceding claims, **characterized in that** the first fastening device (21) can be connected to a foot (5) of a person and the second fastening device (22) can be connected to a lower leg (6) of the person.

## Revendications

1. Orthèse ou prothèse (1) pour la correction d'une mauvaise position d'une articulation reliant deux membres, en particulier d'une cheville, avec un premier dispositif de fixation (21) et un deuxième dispositif de fixation (22), dans laquelle les deux dispositifs de fixation (21, 22) peuvent être fixés respectivement à un premier et à un deuxième des deux membres,
un élément de liaison (3) qui relie les deux dispositifs de fixation (21, 22) l'un à l'autre, dans laquelle
l'élément de liaison (3) présente au moins une barre (30, 31, 32) plate avec deux surfaces principales (30a, 30b, 31a, 31b, 32a, 32b) s'étendant sensiblement parallèlement l'une à l'autre, qui présente sur au moins l'une ou exactement l'une de ses surfaces principales (30a, 30b, 31a, 31b, 32a, 32b) une pluralité de rainures (4) disposées dans l'extension longitudinale de la barre (30, 31, 32) de manière espacée les unes des autres du premier dispositif de fixation (21) au deuxième dispositif de fixation (22), dans laquelle les rainures (4) s'étendent au moins en partie non pas complètement mais par endroits sur la largeur de la barre (30, 31, 32) s'étendant transversalement à la direction d'extension longitudinale et s'étendent suivant un angle W avec 10 °≤ | W | ≤ 90 ° par rapport à l'extension longitudinale de la barre (30, 31, 32), **caractérisée en ce que** les rainures sont disposées sur l'une ou les deux surfaces principales (30a, 30b, 31a, 31b, 32a, 32b) de telle sorte qu'une déviation dans l'une des deux directions perpendiculaires aux deux surfaces principales (30a, 30b, 31a, 31b, 32a, 32b) sensiblement parallèles présente un module d'élasticité différent de celui d'une déviation dans la direction opposée.

2. Orthèse ou prothèse (1) selon la revendication précédente, **caractérisée en ce que** la barre (30, 31, 32) plate comprend une, deux ou plusieurs couches (301, 302, 303) qui s'étendent sensiblement parallèlement à au moins une des surfaces principales (30a, 30b, 31a, 31b, 32a, 32b) de l'au moins une barre (30, 31, 32).

3. Orthèse ou prothèse (1) selon l'une quelconque des revendications précédentes, **caractérisée en ce que** la barre (30, 31, 32) plate présente une largeur C avec 5 mm ≤ C ≤ 35 mm, avantageusement 7 mm ≤ C ≤ 25 mm, avantageusement 10 mm ≤ C ≤ 22 mm.

4. Orthèse ou prothèse (1) selon l'une quelconque des revendications précédentes, **caractérisée en ce que** la barre (30, 31, 32) plate présente une épaisseur D avec 0,5 mm ≤ D ≤ 15 mm, avantageusement 1 mm ≤ D ≤ 10 mm, avantageusement 2 mm ≤ D ≤ 7 mm.

5. Orthèse ou prothèse (1) selon l'une quelconque des revendications précédentes, **caractérisée en ce que** l'au moins une barre (30, 31, 32) plate présente une longueur L avec 20 mm ≤ L ≤ 1000 mm, avantageusement 30 mm ≤ L ≤ 900 mm, avantageusement 50 mm ≤ L ≤ 750 mm.

6. Orthèse ou prothèse (1) selon l'une quelconque des revendications précédentes, **caractérisée en ce que** les rainures (4) s'étendent selon un angle W avec 30° ≤ | W | ≤ 90°, en particulier perpendiculairement à l'extension longitudinale.

7. Orthèse ou prothèse (1) selon l'une quelconque des revendications précédentes, **caractérisée en ce que** les rainures (4) s'étendent au moins par sections en ligne droite et/ou au moins par sections en courbe.

8. Orthèse ou prothèse (1) selon l'une quelconque des revendications précédentes, **caractérisée en ce qu'**au moins une, plusieurs ou toutes les rainures (4) présentent une profondeur T avec 0,3 mm ≤ T ≤ 10 mm, avantageusement 1 mm ≤ T ≤ 6 mm, avantageusement 2 mm ≤ T ≤ 5 mm.

9. Orthèse ou prothèse (1) selon l'une quelconque des revendications précédentes, **caractérisée en ce qu'**au moins une, plusieurs ou toutes les rainures (4) présentent une largeur B avec 0,05 mm ≤ B ≤ 10 mm, avantageusement 0,1 mm ≤ B ≤ 5 mm, avantageusement 0,1 mm ≤ B ≤ 0,5 mm.

10. Orthèse ou prothèse (1) selon l'une quelconque des revendications précédentes, **caractérisée en ce qu'**au moins une, plusieurs ou toutes les rainures (4) présentent les unes par rapport aux autres dans le sens de l'extension longitudinale de la barre (30, 31, 32) une distance A avec 5 mm ≤ A ≤ 40 mm, avantageusement 10 mm ≤ A ≤ 35 mm, avantageusement 12 mm ≤ A ≤ 28 mm.

11. Orthèse ou prothèse (1) selon l'une quelconque des revendications précédentes, **caractérisée en ce que** le premier dispositif de fixation (21) peut être relié à un pied (5) d'une personne et le deuxième dispositif de fixation (22) à une jambe inférieure (6) de la personne.
